(19) Europäisches Patentamt
European Patent Office
Office européen des brevets

(11) Veröffentlichungsnummer: **0 013 766**
**B1**

(12) **EUROPÄISCHE PATENTSCHRIFT**

(45) Veröffentlichungstag der Patentschrift: **11.11.81**

(21) Anmeldenummer: **79105412.5**

(22) Anmeldetag: **31.12.79**

(51) Int. Cl.³: **C 12 P 19/38**, C 12 R 1/465, C 07 H 19/06, C 07 H 19/04// A01N43/50, A01N43/54

# (54) Antibiotica, ein Verfahren zu ihrer Herstellung sowie ihre Verwendung als Pflanzenschutzmittel.

(30) Priorität: **09.01.79 DE 2900591**

(43) Veröffentlichungstag der Anmeldung: **06.08.80 (Patentblatt 80/16)**

(45) Bekanntmachung des Hinweises auf die Patenterteilung: **11.11.81 Patentblatt 81/45**

(84) Benannte Vertragsstaaten: **BE CH DE FR GB IT NL**

(56) Entgegenhaltungen:
**DE - A - 2 537 028**
**DE - A - 2 701 890**

(73) Patentinhaber: **BAYER AG**
**Zentralbereich Patente, Marken und Lizenzen**
**D-5090 Leverkusen 1, Bayerwerk (DE)**

(72) Erfinder: **Zähner, Hans, Dr. Prof.**
**Im Hopfgarten 13**
**D-7400 Tübingen (DE)**
Erfinder: **Holst, Hartwig, Dr.**
**Pestalozzistrasse 1**
**D-6301 Pohlheim 1 (DE)**
Erfinder: **Zoebelein, Gerhard, Dr.**
**Domblick 46**
**D-5090 Leverkusen 31 (DE)**
Erfinder: **Keckeisen, Adelinde**
**Im Philosophen 79**
**D-7400 Tübingen (DE)**

EP 0 013 766 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

# Antibiotica, ein Verfahren zu ihrer Herstellung sowie ihre Verwendung als Pflanzenschutzmittel

Die vorliegende Erfindung betrifft neue Antibiotica, ein mikrobiologisches Verfahren zu ihrer Herstellung aus Streptomyceten-Stämmen sowie ihre Verwendung als Pflanzenschutzmittel.

Es ist bereits bekannt geworden, daß Polyoxine als fungitoxische Mittel breite Anwendung im Pflanzenschutz gefunden haben. Ihr Nachteil besteht jedoch in einer Alkali-Labilität und weiterhin darin, daß sie nur als Gemische wechselnder Zusammensetzung vorkommen und somit schlecht exakt dosierbar und standardisierbar sind.

Ferner ist aus der DE-OS 2 537 028 ein als Nikkomycin bezeichnetes Antibioticum bekannt, das eine starke fungizide Wirkung gegen phytopathogene Pilze aufweist.

Es wurde nun gefunden, daß das aus der DE-OS 2 537 028 bekannte Nikkomycin aus mehreren strukturell unterschiedlichen Komponenten besteht, und daß das Nikkomycin-Gemisch ebenso wie seine Komponenten oder Komponentengruppen als Schädlingsbekämpfungsmittel gegen tierische Schädlinge, insbesondere gegen Insekten und Spinnentiere Wirksamkeit zeigt.

Die erfindungsgemäßen Stoffe werden durch submerse Kultur von geeigneten Mikroorganismen in geeigneten Nährlösungen unter geeigneten physikalischen Bedingungen erzeugt. Sie werden aus der Kulturlösung durch Adsorption und Fällung abgetrennt und durch weitere geeignete Methoden angereichert.

Für das Herstellungsverfahren kann der neue Stamm Streptomyces tendae Ettlinger et al Tü 901 aus der Ordnung der Actinomycetales, Familie Streptomycetaceae, Gattung Streptomyces eingesetzt werden. Dieser Stamm wurde aus einer Bodenprobe von Nikko/Japan isoliert. Er wurde unter der Nr. CBS 354.75 beim Centralbureau voor Schimmelkultures, Baarn/Niederlande (Empfangsbestätgigung für die hinterlegte Kultur: 17. Juli 1975), unter der Nr. ATCC 31160 bei der American Type Culture Collection, Rockville, Maryland/USA am 30. Juni 1975 und unter der Nr. FRI 3136 beim Fermentation Research Institute Osaka/Japan (eingereicht mit Schreiben vom 23. Juni 1975, Empfangsbestätigung für die Hinterlegung in japanisch mit Schreiben vom 7. Oktober 1975 erhalten) hinterlegt. Dieser Stamm gehört zur Gattung Streptomyces und ist durch folgende Eigenschaften gekennzeichnet:

a) Die Sporen sind ellipsoid. Sie haben die Größe von 0,4-0,6 × 1,2-1,4 μ und eine glatte Oberfläche.

b) Die Farbe des Luftmycels ist zu Anfang kreideweiß, im ausgereiften Zustand aschgrau (cinereus).

c) Die Sporenketten sind monopodial verzweigt und in lockeren Schrauben und Schlingen angeordnet.

d) Auf Pepton-Eisen-Agar wurde bei 27 °C ein schwarzes Pigment gebildet. Der Stamm ist chromogen.

Die zusammengefaßten Bestimmungsmerkmale identifizieren den Stamm Tü 901 als zur Art Streptomyces tendae Ettlinger gehörend.

Für das Verfahren zur Herstellung des Nikkomycin-Gemisches verwendet man Nährmedien, die die üblichen Kohlenstoff- und Stickstoffquellen und die notwendigen Salze enthalten. Als Kohlenstoffquelle können verwendet werden: Kohlenhydrate, insbesondere Polysaccharide, wie z.B. Stärke, Disaccharide wie z.B. Maltose und Rohrzucker, Monosaccharide, wie z.B. Glucose und Fruktose. Weiterhin können noch verwendet werden Zukkeralkohole, wie z.B. Mannit und Glycerin, außerdem auch natürlich vorkommende Gemische, wie z.B. Malzextrakt. Als Stickstoffquelle können die üblichen Stickstoffquellen Verwendung finden, so z.B. Eiweißstoffe, Eiweißhydrolysate, Aminosäuren, Ammonium-Ionen, Nitrate, natürlich vorkommende komplexe Stoffe, wie Peptone, Casein-Hydrolysate, «Cornsteep-liquor», Sojamehl, Fleischextrakt und geeignete Mischungen derselben.

Als Hilfsstoffe werden im Nährmedium bevorzugt verwendet die Salze, z.B. Phosphate, Sulfate oder Chloride, des Magnesiums, Eisens, Zinks und Mangans. Die Konzentration dieser Stoffe kann in weiten Grenzen schwanken, zum Teil sind notwendige Konzentrationen in den obengenannten Kohlenstoff- oder Stickstoffquellen oder im verwendeten Wasser als Verunreinigungen enthalten.

Weiterhin können als Hilfsstoffe noch Antischaummittel der verschiedensten Art Verwendung finden, wie z.B. Sojaöl, Polyole oder Silikone. Zur Einhaltung eines gewünschten pH-Bereiches werden Puffer verwendet, auch organische Puffer.

Als wichtigstes Verdünnungsmittel für die Nährmedien ist Wasser zu nennen.

Bei der Durchführung des Herstellungsverfahrens wird im allgemeinen unter aeroben Bedingungen gearbeitet; die Kultur kann gemäß üblichen Methoden, so z.B. unter Verwendung von Schüttelkulturen oder von belüfteten Fermentkulturen, durchgeführt werden. Die Prozentverhältnisse der Nährlösungsbestandteile können in weiten Bereichen schwanken, im allgemeinen machen die Kohlenstoffquellen 1 bis 10 %, vorzugsweise 2 bis 5 % aus, die Stickstoffquellen 0,1 bis 4 %, vorzugsweise 0,5 bis 2 % aus; die Salze liegen in üblichen Konzentrationen vor, vorzugsweise im Bereich zwischen 0,01 bis 1 Gewichtsprozent. Die Antischaummittel liegen in 0- bis 1-%iger Konzentration vor. Die zur Sterilisation angewandten Temperaturen liegen bei 100 bis 140 °C, vorzugsweise bei 120 bis 130 °C.

Die pH-Werte der wachsenden Kulturen liegen bei 5,5 bis 8, vorzugsweise bei 7 bis 7,5. Die Züchtungstemperatur kann zwischen 18 und 37 °C, vorzugsweise bei 27 bis 30 °C liegen. Es hat sich herausgestellt, daß die Menge des sich in der Kulturbrühe anreichernden Antibioticums im allgemeinen ihr Maximum etwa 1 bis 14, vorzugsweise etwa 3 bis 5 Tage nach Züchtungsbeginn erreicht. Der

Endpunkt der Bebrütung wird mit Hilfe von biologischen Tests ermittelt, und zwar wird die Wirkung gegen Botrytis cinerea (Testverfahren nach R. Hütter et al, Arch. Mikrobiol. *51,* 1-8 [1965]) und Mucor hiemalis (Testverfahren nach Dissertation G. Kirst, Tübingen [1971]), ferner nach Kneifel et al, J. Antib. A *27,* 20-27 [1974] ermittelt.

Bei der Durchführung des Herstellungsverfahrens kann zur Aufarbeitung der Kulturlösungen zunächst eine Filtration durchgeführt werden, wobei das Mycel abgetrennt wird. Letzteres kann der Ionenaustausch-Chromatographie an geeigneten Austauschern unterworfen werden. Die Chromatographie kann in Form der Säulenchromatographie oder der präparativen Dünnschichtchromatographie durchgeführt werden. Als Adsorptionsmittel können alle üblichen (nicht sauren) anorganischen oder organischen Adsorptionsmittel eingesetzt werden, wie z.B. Aluminiumoxid, Kieselgel, Magnesiumsilikat, Aktivkohle, Cellulose, Cellulose-derivate, Kunstharze wie Polyamide, Derivate von Polyamiden usw., z.B. acetyliertes Polyamid oder Dextrangele. Als Laufmittel bei der präparativen Dünnschichtchromatographie können die verschiedensten Lösungsmittel oder Lösungsmittelgemische verwendet werden, in welchen das erfindungsgemäße Antibioticum löslich ist. Anschließend kann eine Gel-Chromatographie und eine Reinisolierung an einer weiteren Säule mit anschließender Gefriertrocknung durchgeführt werden.

Das Nikkomycin-Gemisch, das Gegenstand der DE-OS 2 537 028 ist, kann durch folgende Angaben charakterisiert werden:

a) Löslichkeit und Eigenschaften: Nikkomycin ist ein farbloses, in Wasser und Pyridin sehr gut lösliches, in den sonstigen üblichen organischen Lösungsmitteln unlösliches Substanzgemisch. Es zeigt eine positive Reaktion mit Ninhydrin, Natriummetaperjodat-Benzidin und Kaliumpermanganat. Mit Eisen-(III)-chlorid erhält man eine Gelbfärbung.

b) Das UV-Spektrum und das IR-Spektrum liegen vor (vgl. die entsprechenden Darstellungen in Figuren 1 und 2). In Figur 1 ist das UV-Spektrum von Nikkomycin, aufgenommen in (a) 1 n-Salzsäure und (b) in 0,1 n-Natronlauge, aufgetragen.

Die Ordinate gibt die Extinktion, die Abszisse die Wellenlänge (nm-Einheiten) wieder. In Figur 2 ist das IR-Spektrum von Nikkomycin, aufgenommen in Kaliumbromid, angegeben. Die Ordinate zeigt die Durchlässigkeit in %, die Abszisse die Wellenzahl ($cm^{-1}$) bzw. Wellenlänge (μm-Einheiten) auf.

c) Mit Hilfe der Massenspektrometrie und des chemischen Abbaus durch saure Hydrolyse konnten Uracil, eine Amino-hexuronsäure und eine neue, einen Pyridinring enthaltende Aminosäure nachgewiesen werden.

d) Papierelektrophorese: Das Antibioticum ist amphoter. Um den pH-Wert von 6 ist die Wanderungsstrecke klein, hier dürfte somit der isoelektrische Punkt liegen. Das Verhalten des Nikkomycin-Gemisches bei der Elektrophorese zeigt die nachfolgende Tabelle, in welcher die Laufstrecken bei der Papierelektrophorese in Abhängigkeit von den pH-Werten des Puffersystems angegeben sind.

| Puffer | pH | Zeit (min) | Laufstrecke (mm) |
|---|---|---|---|
| Pyridin-Eisessig | 3,9 | 60 | − 12 |
| Pyridin-Eisessig | 6,1 | 60 | − 1 |
| Barbital (*) | 8,9 | 60 | + 16 |

(*) Barbital ist 5,5-Diäthyl-Barbitursäure

e) Dünnschichtchromatographie: Hierbei treten in allen verwendeten Fließmitteln, die Essigsäure enthalten, drei ninhydrin-positive Flecken auf, wobei jeweils nur ein Fleck mit der im Bioautogramm aktiven Substanz korrespondiert. Diese Tatsache konnte auf die Labilität des Antibioticums in essigsaurer Lösung zurückgeführt werden. In neutralen Fließmitteln zeigt sich jeweils nur ein ninhydrin-positiver Fleck.

f) Als Strukturformel für Nikkomycin wird vorgeschlagen:

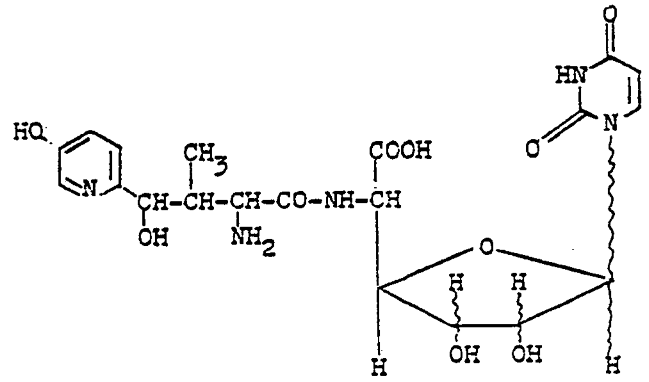

Die Elementaranalyse von Nikkomycin ergab folgende Werte: C 48,21; H 5,08; N 13,58; O 31,58. Daraus errechnet sich eine Elementarzusammensetzung von $C_4H_5NO_2$. Kryoskopisch wurde das Molekulargewicht mit 404 bestimmt. Die hier vorgeschlagene Struktur für Nikkomycin entspricht jedoch dem Molekulargewicht von 495 und der Formel $C_{20}H_{25}N_5O_{10} = (C_4H_5NO_2)_5$. Die daraus errechnete Elementarzusammensetzung ergibt:

C 48,49 %; H 5,09 %; N 14,14 %; O 32,29 %.

g) Im Gegensatz zu den literaturbekannten Polyoxinen (vgl. J. Am. Chem. Soc. *91,* 7490 [1961]), welche dem Nikkomycin am nächsten stehen, fehlt im Nikkomycin der Azacyclobutan-Rest. Die heterocyclische Aminosäure ist in keinem der beschriebenen Polyoxine enthalten.

Die Charakteristik entspricht den Angaben in der DE-OS 2 537 028. Sie gilt wie inzwischen gefunden wurde für ein Gemisch strukturell unterschiedlicher, bisher nicht beschriebener Nikkomycine. Demgemäß betrifft die Erfindung antibiotische Nikkomycine der Formel

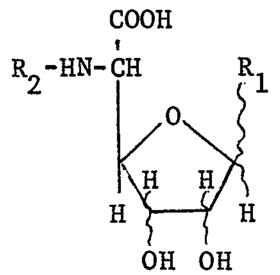

in der $R_1$ die Bedeutung

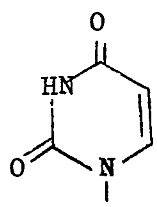

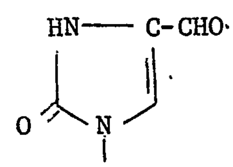

und $R_2$ die Bedeutung Wasserstoff,

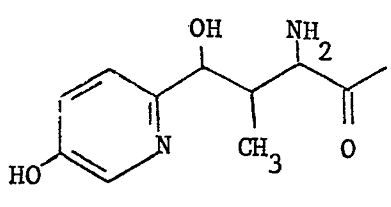

oder

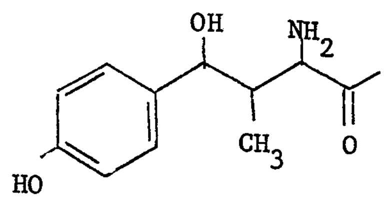

hat

mit der Maßgabe, daß falls
$R_1$ die Bedeutung

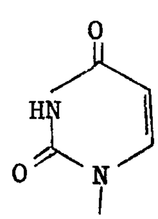

hat,

$R_2$ nicht

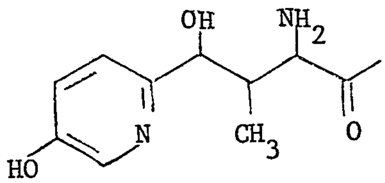

sein kann, sowie

deren Verwendung als Schädlingsbekämpfungsmittel.

Bevorzugt betrifft die Erfindung die folgenden Wirkstoffkomponenten bzw. Wirkstoffkomponentenpaare sowie deren Verwendung als Schädlingebekämpfungsmittel

a)

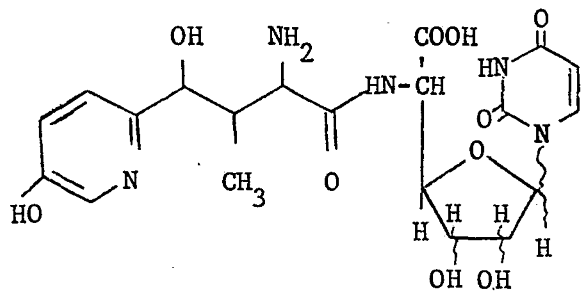

(Z) und

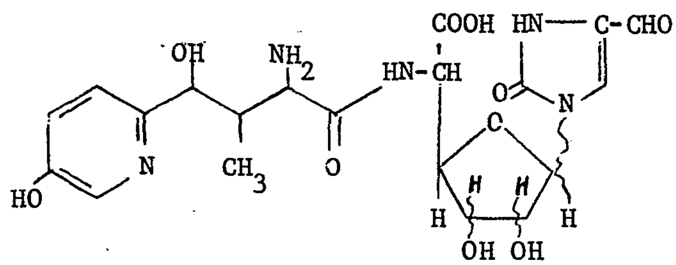

(X)

b)

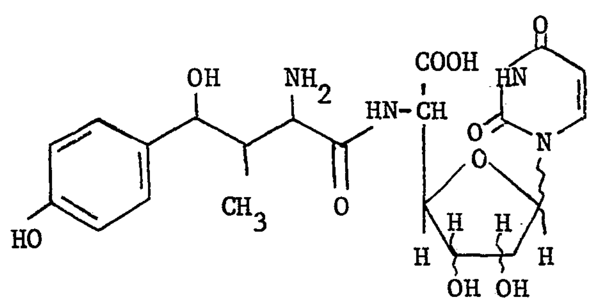

(B) und

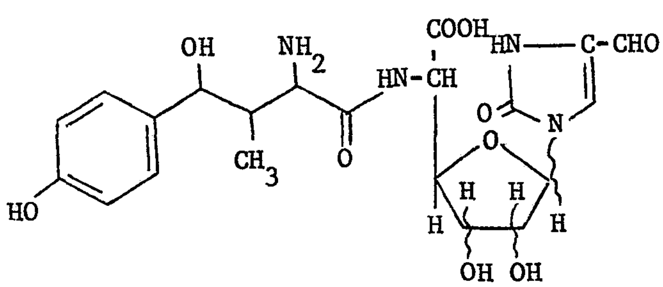

($B_x$)

c) 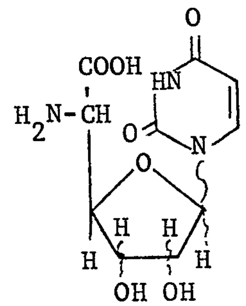 (c)

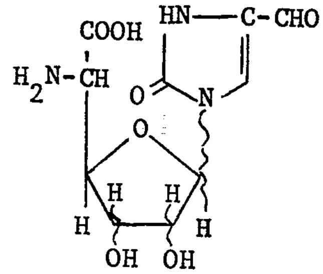 ($c_x$)

Das Nikkomycin-Gemisch und seine Komponenten bzw. Komponentenpaare zeigen überraschenderweise eine ausgezeichnete Wirkung gegen tierische Schädlinge insbesondere gegen Insekten und Spinnentiere bei guter Pflanzenverträglichkeit und günstiger Warmblütertoxizität.

Die erfindungsgemäßen Wirkstoffe zeigen bei Insekten eine fraßabschreckende, entwicklungsverzögernde und -hemmende Wirkung. Bei Spinnmilben wird durch Anwendungen von Nikkomycin-Gemischen bzw. Nikkomycinkomponenten die langsame Ausrottung einer Population bereits nach wenigen Behandlungen erreicht. Insbesondere erfolgt je nach Anwendungszeitpunkt eine fast gänzliche Hemmung der Weiterentwicklung in den Ruhestadien Nymphochrysalis, Deutochrysalis und Teleiochrysalis, ein Effekt, der bisher von anderen Naturstoffen oder mikrobiellen Metaboliten nicht bekannt ist. Ein Zusatz anderer Wirkstoffe mit synergistischer oder additiver Wirkung ist nicht erforderlich. Nikkomycin ist auch gegen phosphorsäureesterresistente Spinnmilben hervorragend wirksam. Die niedrige orale Toxizität für Säugetiere (> 2 000 mg/kg LD 50 bei der Ratte) ist ein weiterer Vorteil.

## Beispiel A

Langzeitversuch mit Pieris brassicae (Kohlweißling)

Behandelte Kohlpflanzen wurden in einen Käfig aufgestellt und mit Raupen des 2. Stadiums besetzt. Der Versuch wurde bei Verpuppung aller Raupen in unbehandelt (nach 15 Tg.) abgebrochen. Entwicklungsverzögerung und abtötende Wirkung:

| % Präparat | | | | % Mortalität nach 15 Tg. |
|---|---|---|---|---|
| 0,025 : | 0 Puppen | 0 Vorpuppe | 6 Larven | 63 |
| 0,01 : | 0 Puppen | 3 Vorpuppen | 5 Larven | 50 |
| Kontrolle : | 13 Puppen | 2 Vorpuppen | 1 Larve | 0 |

## Beispiel B

Langzeitversuch mit Laphygma exigua (Heerwurm)

Versuchsanwendung wie Beispiel A

Eine abtötende Wirkung trat nicht ein, dagegen wurde durch die Präparateeinwirkung die Entwicklungsdauer von der $L_2$-Larve bis zum Falter um 1-3 Tage verlängert, das Puppengewicht um 5-10 %, die Eiproduktion um ca. 50 % reduziert.

## Beispiel C

Langzeitversuch mit Myzus persicae (Grüne Pfirsichblattlaus)

Bei gleicher Startpopulation wurde der Vermehrungsgrad $= \frac{\text{Anzahl der Tiere nach 9 Tagen}}{\text{Anzahl der Tiere nach 2 Tagen}}$

untersucht. Bei 0,1 % Präparat beträgt er 1,9 und ist demnach gegenüber der Kontrolle mit 2,4 geringfügig herabgesetzt.

Beispiel D

Langzeitversuch mit Megoura viciae (Wickenblattlaus)

Die Untersuchungen mit Megoura viciae wurden an getopften Ackerbohnenpflanzen (Vicia faba, Sorte «Saxa») unter Laborbedingungen durchgeführt. Die Versuchstiere wurden erst nach der Pflanzenbehandlung und dem Antrocknen des Spritzbelages auf die Versuchspflanzen übertragen. Die Auswertung der Versuche erfolgte in der Regel 5 Tage nach Versuchsbeginn, vom Zeitpunkt des Ansetzens der Versuchstiere an gerechnet.

Wie die Ergebnisse der Tabelle 1 zeigen, wurde die Mortalität adulter Tiere durch Nikkomycin nicht sehr stark beeinflußt, während die Vermehrung gemessen an der Anzahl abgesetzter Larven/Weibchen und Tag um 40 % durch 1 000 und 500 ppm verringert wurde. Besonders auffällig war die hohe Mortalität bei den abgesetzten Larven. 1 000 ppm verhinderten vollständig die Entwicklung einer neuen Population. Fast 60 % der abgesetzten Larven waren nicht lebensfähige Tiere, die in den Tabellen als «amorphe» Larven bezeichnet wurden. Die Tiere wiesen teilweise so starke morphogenetische Schäden auf, daß sie kaum noch als Larven angesprochen werden konnten. Auch mit 500 und 100 ppm wurden noch sehr gute Ergebnisse erzielt.

Die Mortalität bei den normal entwickelten Larven tritt zu über 90 % während der Häutungsschritte, besonders vom 2. und 3. Larvenstadium auf. Die Larven waren aufgrund der morphogenetischen Störungen (unvollständig entwickelte Gliedmaßen oder miteinander verklebte oder verwachsene Gliedmaßen) nicht in der Lage, aus der Exuvie zu schlüpfen.

Tabelle 1

Wirkung von Nikkomycin auf die Mortalität und Vermehrung von Megoura viciae sowie die Lebensfähigkeit der Larven während 5 Untersuchungstagen.

| Konzentration in ppm | Mortalität adulter Tiere (%) | Gesamtvermehrungsrate (%) | Vermehrungsrate L/A/Tag | Larvenmortalität (%) | amorphe Larven (%) |
|---|---|---|---|---|---|
| 1 000 | 12,50 | 53,60 | 3,0 | 100 | 59,57 |
| 500 | 9,37 | 68,91 | 3,9 | 99,27 | 55,43 |
| 100 | 19,66 | 84,60 | 4,8 | 92,65 | 42,26 |
| 10 | 19,23 | 82,70 | 4,7 | 5,23 | 0 |
| Kontrolle | 16,22 | 100 | 5,7 | 0,99 | 0 |

Beispiel E

Stadienversuche mit Tetranychus urticae (Gemeine Bohnenspinnmilben)

Bohnenpflanzen wurden mit jeweils verschiedenen Stadien Spinnmilben der Art Tetranychus urticae (Phosphorsäureester-resistent) besetzt und ihre Entwicklung alle 2-3 Tage kontrolliert. Folgende Stadien wurden zur Prüfung verwendet und auf Bohnenpflanzen behandelt: Adulte ♂♂ und ♀♀, Eier und bewegliche Larvenstadien.

Bei Dosierungen zwischen 0,02 und 0,004 % des nichtformulierten Wirkstoffs wurde die Entwicklung jeweils bei dem folgenden Ruhestadium (Nymphochrysalis, Deutochrysalis oder Teleiochrysalis) gestoppt.

Beispiel F

Vergleich der Wirkung an normal sensiblen und resistenten Tetranychus urticae

Bohnenpflanzen mit Eiern eines normal sensiblen Stammes und eines phosphorsäureesterresistenten Stammes von Tetranychus urticae wurden mit verschiedenen Dosierungen von Nikkomycin behandelt, die Weiterentwicklung alle 2-3 Tage bis zum Erreichen des Adultenstadiums in der unbehandelten Kontrolle beobachtet. Bei 0,02-0,004 % Wirkstoffkonzentration verlief die Entwicklung bei normal sensiblen und resistenten Spinnmilben in gleicher Weise bis zum Nymphochrysalisstadium, aus dem dann keine Protonymphen schlüpften.

Beispiel G

Freilandversuch gegen Panonychus ulmi (Obstbaumspinnmilbe)

Um die im Labor gefundenen Wirkungen von Nikkomycin im Freiland und an einer anderen

Spinnmilbenart zu bestätigen, wurden jeweils zwei Apfelbäume mit verschiedenen Konzentrationen von Nikkomycin unter Zusatz eines Netzmittels behandelt. Auf den Blättern befanden sich zum Behandlungszeitpunkt alle Stadien von Panonychus ulmi. Durch drei Spritzungen mit 0,005 % Nikkomycin-Wirkstoff wurde die Anzahl überlebender beweglicher Stadien um 94 % im Vergleich zur unbehandelten Kontrolle verringert.

Herstellung des Nikkomycin-Gemisches

Die Nährlösung, in der der Produzentenstamm Streptomyces tendae TÜ 901 kultiviert wird, setzt sich aus 2 % Sojamehl und 2 % Mannit zusammen; der pH wird vor dem Sterilisieren auf pH 7,5 eingesetzt. 10 × 500-ml-Erlenmeyerkolben mit 1 seitlichen Einstich, die je 100 ml Nährlösung enthalten, werden mit dem Produzentenstamm beimpft und 48 Stunden bei 27 °C auf einer rotierenden Schüttelmaschine bei 120 Umdrehungen/Minute inkubiert. Mit dieser Vorkultur wird ein 10 l-Fermenter («New Brunswick»), der 10 l Nährlösung enthält, beimpft, bei 220 Umdrehungen/Minute, 4 l Luftzufuhr/Minute und 27 °C 48 Stunden inkubiert. Mit diesem Vorfermenter wird ein 100 l-Fermenter («New Brunswick»), der 100 l Nährlösung enthält, beimpft, bei 150 Umdrehungen/Minute, 450 l Luftzufuhr/Minute und 27 °C 78 Stunden inkubiert.

Die Kultur wird unter Zugabe von 2 % Filterhilfsmittel (Hyphlo Supercel(R), Johns Mansville) mit einer Filterpresse zuerst über ein Vorklärfilter (C 150, Schenk) und danach über ein Nachklärfilter (U 1000, Schenk) abgepreßt. Das klare Kulturfiltrat wird mit Essigsäure auf pH 4,0 angesäuert und auf eine mit Dowex 50 W × 4(R) (50-100 mesh, $Na^+$-Form) gefüllte Säule (100 × 450 mm) aufgetragen. Die Fließgeschwindigkeit beträgt 10 l/Stunde. Es wird so lange mit entionisiertem Wasser gewaschen, bis die aus der Säule austretende Flüssigkeit völlig farblos ist. Das Antibioticum wird mit je 30 l 0,01 n-Ammoniak und 0,05 n-Ammoniak eluiert. Das biologisch aktive Eluat wird am Rotationsverdampfer von Ammoniak befreit, mit Essigsäure auf pH 4,0 angesäuert und auf eine mit «Amberlite 252»(R) ($Na^+$-Form) gefüllte Säule (70 × 900 mm) aufgetragen. Die Fließgeschwindigkeit beträgt 5 l/Stunde. Es wird so lange mit entionisiertem Wasser gewaschen, bis die aus der Säule austretende Flüssigkeit völlig farblos ist. Das Antibioticum wird mit 15 l 0,05 n-Ammoniak eluiert. Das biologisch aktive Eluat wird am Rotationsverdampfer vom Ammoniak befreit, auf ein kleines Volumen eingeengt (1 l), mit Essigsäure auf pH 4,0 angesäuert und auf eine mit SP-Sephadex C-25 gefüllte Säule (25 × 850 mm) aufgetragen. Die Fließgeschwindigkeit beträgt 100 ml/Stunde. Mit entionisiertem Wasser wird so lange gewaschen, bis im UV-Durchfluß-Detektor bei 280 nm (Uvicord II, LKB) keine Absorption mehr angezeigt wird. Mit 0,01 m-Pyridin-Acetat-Puffer (pH 4,7) werden Verunreinigungen und mit 0,02 m-Pyridin-Acetat-Puffer (pH 4,7) wird das Antibioticum eluiert. Die biologisch aktiven Fraktionen werden vereinigt, am Rotationsverdampfer vom Puffer befreit und auf ein sehr kleines Volumen eingeengt. Das Konzentrat (10 ml) wird auf eine mit Bio-Gel P 2 (200-400 mesh) gefüllte Säule (25 × 1 500 mm) aufgetragen und mit entionisiertem Wasser eluiert. Die Fließgeschwindigkeit beträgt 100 ml/Stunde. Zur Reinheitskontrolle wird mit einem UV-Durchfluß-Detektor bei 280 nm detektiert. Die biologisch aktiven Fraktionen werden vereinigt und in einer Gefriertrocknungsanlage lyophilisiert.

Die Isolierung der erfindungsgemäßen Komponenten aus dem so hergestellten Nikkomycin-Gemisch wird im folgenden am Beispiel der Nikkomycine X und Z gezeigt.

Isolierung von Nikkomycin Z

100 mg Nikkomycin wurden mit 10 ml einer gesättigten, wäßrigen Dimedonlösung versetzt und 10 Min. auf dem Wasserbad bei 50-70 °C erwärmt und anschließend 20 h bei Raumtemperatur stehengelassen. Nach Eindampfen am Rotationsverdampfer wurde der Rückstand in 1 ml $H_2O$ aufgenommen und an einer Säule (0,9 × 60 cm) mit Cellulose (Avicel, Fa. Merck) mit dem Laufmittelsystem A chromatographiert. Das orange-rote Dimedonderivat von X wird zusammen mit überschüssigem Dimedon vor Z eluiert. Die Fraktionen mit Z wurden vereinigt und an derselben Säule rechromatographiert.

Ausb. 11,4 mg Z.

Isolierung von Nikkomycin X

19 g Nikkomycin wurden in 75 ml $H_2O$ gelöst und 9 g Alkylamine/CPG-1350 (Pierce Chem. Comp.) zugegeben. Nach dem Entgasen im Vakuum ließ man 24 h bei Raumtemperatur stehen. Das Trägermaterial wurde abfiltriert, mit 800 ml $H_2O$ ausgewaschen und mit 100 ml 5 % Essigsäure 30 Min. bei Raumtemperatur behandelt. Das Filtrat wurde lyophilisiert. Man erhielt einen Rückstand von 116 mg, der anschließend an LiChroprep RP-8 mit Methanol/$H_2O$ (30 : 70) chromatographiert wurde. Ausbeute an X (nach Lyophilisieren) 18 mg.

Bestimmung des Verhältnisses von Nikkomycin X und Z

a) Mittels $^1H$-NMR-Spektroskopkie Nikkomycin Z läßt sich im $^1H$-NMR-Spektrum durch die beiden miteinander koppelnden Protonen in Stellung 5 ($\delta = 5,91$; d; 8 Hz) und Stellung 6 ($\delta = 7,59$ ; d; 8 Hz)

erkennen. Im Nikkomycin X fehlen diese beiden Resonanzen. Dafür treten zwei Singuletts bei δ = 7,69 und δ = 9,33 auf. Durch Vergleich der Integrale dieser Signale ist eine Bestimmung des Verhältnisses von Nikkomycin X und Z in der Mischung möglich.

b) Mittels Hochdruckflüssigkeitschromatographie An einer RP-18 Säule (Merck, 10 u. 250 × 4,6 mm) mit einer 0,005 molaren Lösung von 1-Hexansulfonsäure in Methanol/$H_2O$/Essigsäure (14/84/2) als Elutionsmittel lassen sich die beiden Nikkomycine X und Z auftrennen und mittels UV-Detektion bei 280 nm quantitativ bestimmten. Diese Bestimmung kann direkt aus dem Kulturfiltrat erfolgen.

Im obigen Herstellungsbeispiel werden, zum Teil mit Warenzeichenbezeichnung, Reagenzien, Hilfsmittel und technische Geräte folgender Firmen genannt:

a) Fermenter der Firma New Brunswick Scientific Corporation Inc., New Brunswick, New Yersey/USA
b) Hyphlo Supercel$^{(R)}$, Firma Johns, Mansville, Cal., USA
c) Filterpressen C 150 und U 1000 der Firma Schenck, Filterbau, Schwäbisch-Gmünd, BRD
d) Dowex$^{(R)}$, Warenzeichen der Firma Dow Chemical Co., Midland, Michigan/USA
e) Amberlite$^{(R)}$, Warenzeichen der Firma Rohm und Haas Co., Philadelphia, Pennsylvania/USA
f) SP-Sephadex$^{(R)}$, Warenzeichen der Firma Pharmacia Fine Chemicals, Upsala/Schweden
g) Uvicord$^{(R)}$ II, Firma LKB, Bromma, Schweden.

Eigenschaften der Nikkomycine Z und X

Beide Nikkomycine fallen nach dem Lyophilisieren als voluminöse farblose Substanzen an. Sie sind beide gut löslich in Wasser, Pyridin und Dimethylsulfoxid, aber schlecht löslich in Methanol, Äthanol, Chloroform und Äther. Beide geben eine positive Reaktion mit Ninhydrin, Chlor-Tolidin, Natriummetaperjodat, Folin-Ciocalteau's Reagens (positiv auf Hydroxypyridylhomothreonin in Z und X und auf 4-Formyl-4-imidazolin-2-on in X) und Kaliumpermanganat. Nikkomycin X reagiert im Gegensatz zu Z aufgrund seiner Aldehydgruppe mit 2,4-Dinitrophenylhydrazin, p-Nitrophenylhydrazin, Dimedon und Benzidin.

Dünnschichtchromatographische Untersuchung der Nikkomycine Z und X

Um die Nikkomycine Z und X auf Dünnschichtplatten zu trennen, wurde die Z, X-Mischung mit essigsaurer p-Nitrophenylhydrazinlösung oder wäßriger Dimedonlösung behandelt, Um Nikkomycin X zu derivatisieren.

Das p-Nitrophenylhydrazon und das Dimedonderivat von Nikkomycin X unterscheiden sich in ihren Rf-Werten von Nikkomycin Z.

| Substanz | Rf |
|---|---|
| Nikkomycin Z | 0,27 |
| Nikkomycin X | 0,27 |
| p-Nitrophenylhydrazon von Nikkomycin X | 0,35 |
| Dimedonderivat von Nikkomycin X | 0,73 |

Rf-Werte von Nikkomycin Z, Nikkomycin X und Nikkomycin X-Derivaten im Fließmittel Äthanol/Essigsäure/Wasser (4 : 1 : 1) auf Cellulose Fertigplatten (Merck, Darmstadt, Deutschland).

Die Umsetzung von Nikkomycin X erfolgte unter den gewählten Bedingungen quantitativ — der Fleck mit Rf = 0,27 war anschließend nicht mehr mit 2,4-Dinitrophenylhydrazin anfärbbar.

$^1$H-NMR-Spektren der Nikkomycine Z und X

Die beiden Spektren der beiden Verbindungen unterscheiden sich nur im Basenanteil. Bei Nikkomycin Z sind zwei Dubletts vorhanden, von denen das bei 7,54 ppm dem Proton H-6 und das bei 5,78 ppm dem Proton H-5 des Uracils entspricht. Die Kopplungskonstante beträgt 8,06 Hz, was größenordnungsmäßig bei Uracilderivaten üblich ist. Im Gegensatz dazu weist Nikkomycin X zwei Singuletts auf, bedingt durch das Vorhandensein von 4-Formyl-4-imidazolin-2-on. Das Aldehydproton erscheint bei 9,28 ppm und das Proton H-5 bei 7,76 ppm. H-1′ des Zuckeranteils von Nikkomycin Z ist bei 5,76 ppm (J = 5,13 Hz) als Dublett zu erkennen, überlagert vom Uracildublett. Bei Nikkomycin X ist die Resonanz von H-1′ bei 5,63 ppm (J = 5,13 Hz). Die übrigen Zuckerprotonen lassen sich nicht näher zuordnen. Man kann ersehen, daß beide Nikkomycine dieselbe Aminosäure enthalten. Die Methylgruppe ergibt ein Dublett bei 0,67 bzw. 0,79 ppm H-2″ kommt zusammen mit den Zuckerprotonen. Das Multiplett bei 2,63 ppm wird durch H-3″ verursacht, welches mit H-2″, H-4″ und H-5″ koppelt. H-4″ gibt ein Dublett bei 5,02 bzw. 5,13 ppm. Von den aromatischen Protonen des Pyridinrings hat H-8″ eine Resonanz bei 7,98 bzw. 8,06 ppm und H-10″, 11″ und bei 7,35 bzw. 7,45 ppm. Die in beiden Spektren auftauchende Essigsäure stammt aus der Aufarbeitung.

UV-Spektren der Nikkomycine Z und X

Das UV-Spektrum von Nikkomycin Z zeigt in 0,01 N HCl ein Maximum bei 257 nm (ε = 7 300) und eine Schulter bei 289 nm (ε = 4 600). In 0,01 N NaOH sind Maximum bei 244 nm (ε = 10 700) und 301 nm (ε = 3 500) und eine Schulter bei 270 nm (ε = 4 700) vorhanden.

Nikkomycin X hat in 0,01 N HCl ein Maximum bei 286 nm (ε = 14 500). Dieses verschiebt sich in 0,01 NaOH nach 303 nm (ε = 9 300), und bei 241 nm (ε = 14 000) erscheint ein zweites Maximum.

IR-Spektren der Nikkomycine Z und X

Die Spektren der Verbindungen sind sehr ähnlich. Stark ausgeprägt ist die Bande bei 1 660 (Nikkomycin Z) bzw. 1 630 μ (Nikkomycin X), bedingt vor allem durch C = 0 Valenzschwingungen. Weiterhin fällt eine breite Bande zwischen 3 000 und 3 500 μ auf.

Eigenschaften der Nikkomycine CX und C

Beide Stoffe fallen nach der Gefriertrocknung als voluminöse farblose Substanzen an. Sie sind gut löslich in heißem Wasser, Pyridin und Dimethylsulfoxid, aber schlecht löslich in Methanol, Äthanol, Chloroform und Äther.

Beide Verbindungen geben eine positive Reaktion mit Ninhydrin, Chlor-Tolidin, Natriummetaperjodat und Kaliumpermanganat. Verbindung CX bildet zusätzlich mit 2,4-Dinitrophenylhydrazin und p-Nitrophenylhydrazin tiefgelbe Hydrazone. 2,4-Dinitrophenylhydrazin ist zwar ein Carbonyl-Reagens, aber auch andere Substanzen wie z.B. Pyrrol, Phenazin, einige Phenole und Indolderivate geben eine Farbreaktion. Aus Dimedon, welches bei Raumtemperatur ein spezifisches Aldehydreagens ist, und Verbindung CX entsteht ein orangerotes Kondensationsprodukt.

Verbindung CX gibt auch mit Benzidin eine Gelbfärbung, die auf die Bildung einer Schiff'schen Base zurückzuführen ist. Mit Folin-Ciocalteau's Reagens entsteht bei dieser Verbindung eine Blaufärbung.

Dünnschichtschromatographische Untersuchung der Nikkomycine CX und C

Bei der Dünnschichtchromatographie werden folgende Werte erhalten (Fließmittel und Platten wie bei Nikkomycin Z und X) :

Nikkomycin CX : Rf = 0,11

Nikkomycin C : Rf = 0,09.

$^1$H-NMR-Spektren der Nikkomycine CX und C

Die $^1$H-NMR-Spektren der Verbindung wurden in $D_2O$ aufgenommen, wobei alle NH- und OH-Protonen ausgetauscht wurden. Im Spektrum der Verbindung CX sind zwei Singuletts bei 9,33 und 7,69 ppm zu erkennen, die der Nucleobase zuzuordnen sind. Das bei 9,33 ppm weist auf ein Aldehydproton und das bei 7,69 ppm auf ein aromatisches Proton hin. Die vom Zuckeranteil hervorgerufenen Protonenresonanzen sind bei Verbindung CX sehr gut aufgelöst und können mit Hilfe von Entkopplungsexperimenten wie folgt interpretiert werden: Das einem Proton entsprechende Dublett bei 4,22 ppm (J = 2,4 Hz) ist dem Proton H-5' zuzuordnen. Bei 4,44 ppm erscheint ein Multiplett, das die Resonanzen der Protonen H-2' und H-4', die jeweils mit den benachbarten Protonen koppeln, wiedergibt. Das Proton H-3' erscheint als Triplett bei 4,68 ppm (J = 5,4 Hz ; durch Rotationsseitenbande des HDO-Signals überlagert). Das Dublett bei 5,64 ppm (J = 5,4 Hz) entspricht dem anomeren Proton H-1'.

Das $^1$H-NMR-Spektrum der Verbindung C läßt die Signale der beiden miteinander koppelnden Protonen des Uracilrestes erkennen. Das Dublett bei 5,91 ppm (J = 8 Hz) ist dem Proton in Stellung 5 und das bei 7,59 ppm (J = 8 Hz) dem Proton in Stellung 6 zuzuordnen[(44)].

Das Dublett bei 5,81 ppm (J = 4 Hz) entspricht dem anomeren Proton H-1' des Aminouronsäureanteils. Die weiteren Resonanzen dieses Molekülteils erscheinen als nicht aufgelöste Multipletts bei 4,37 ppm (entsprechend 3 H) und 4,67 ppm (entsprechend 1 H). Das Spektrum zeigt außerdem drei schwache Signale bei 9,33 ppm, 7,69 ppm und 5,64 ppm, die auf eine geringe Beimengung von Verbindung $C_x$ hinweisen.

UV-Spektren der Nikkomycine CX und C

Das UV-Spektrum der Verbindung CX zeigt in 0,01 N HCl ein Absorptionsmaximum bei 287 nm (ε = 16 500), welches sich in 0,01 N NaOH nach 307 nm (ε = 12 800) verschiebt.

Bei Verbindung C ist in 0,01 n HCl ein Maximum bei 262 nm (ε = 7 600), das sich in 0,01 N NaOH nur geringfügig nach 264 nm (ε = 5 100) verschiebt.

Isolierung der Nikkomycine B/$B_x$ und C/$C_x$

Klares Kulturfiltrat wird auf eine Dowex 50WX4 (50-100 mesh $Na^+$-Form) gefüllte Säule aufgetragen (Bettvolumen 31, Fließgeschwindigkeit 7 l/h). Mit entionisiertem Wasser wäscht man solange, bis die aus der Säule austretende Flüssigkeit völlig farblos ist. Verunreinigungen werden mit 0,01 N $NH_3$-Lösung und das Nikkomycingemisch mit 0,05 N $NH_3$-Lösung eluiert. Das biologisch aktive Eluat wird im Vakuum vom Ammoniak befreit und mit Ameisensäure auf pH 5,5 angesäuert.

Das so behandelte Eluat wird auf eine mit Amberlite IRC-84 ($Na^+$-Form) gefüllte Säule aufgetragen (Bettvolumen 2 l, Fließgeschwindigkeit 5 l/h). Die Säule wird mit der dreifachen Menge des Auftragsvolumens mit Wasser gewaschen. Der Durchlauf und das Waschwasser der Amberlite IRC-84 Säule werden vereinigt und auf eine Dowex 60WX4 ($Na^+$-Form, 50-100 mesh) gefüllte Säule aufgetragen (Bettvolumen 700 ml, Fließgeschwindigkeit 3 l/h). Mit 0,01 N $NH_3$-Lösung werden Verunreinigungen und mit 0,03 N $NH_3$-Lösung das Nikkomycingemisch eluiert. Die aktiven Eluate werden vereinigt, am Rotationsverdampfe vom Ammoniak befreit, der pH mit Essigsäure auf 4,7 eingestellt und auf eine mit SP-Sephadex C-25 gefüllte Säule (Bettvolumen 500 ml, Fließgeschwindigkeit 100 ml/h) aufgetragen. Die Säule wird mit entionisiertem Wasser solange gewaschen, bis im UV-Detektor bei 280 mm keine Absorption mehr angezeigt wird. Im Durchlauf und im Waschwasser sind die Nikkomycine B/$B_x$ und C/$C_x$ enthalten. Der Durchlauf und das Waschwasser werden vereinigt, auf ein sehr kleines Volumen eingeengt und über Biogel P2 (50-100 mesh), Bettvolumen 1 l, Fließgeschwindigkeit 150 ml/h) chromatographiert. Man erhält eine Auftrennung in Nikkomycin C/$C_x$ und B/$B_x$.

Überlappende Fraktionen werden rechromatographiert. Die weitere Reinigung der Nikkomycine

erfolgt dann durch Gelchromatographie an Biogel P2 (100-200 mesh, Bettvolumen 500 ml, Fließgeschwindigkeit 80 ml/h). Zur Gewinnung von Reinsubstanz wird eine Reversed-phase-chromatographie an LiChroPrep RP8 (Korngröße 20-40 μ, Bettvolumen 250 ml, Fließgeschwindigkeit 60 ml/h) durchgeführt. Als Laufmittel dient entionisiertes Wasser.

Die weitere Auftrennung kann nach den üblichen Methoden erfolgen.

Dünnschichtchromatographie :

Träger : Cellulose-Fertigplatten, Merck, Darmstadt, Deutschland

Laufmittel : Propanol/Wasser 8 : 2

Mischung B/$B_x$ Rf = 0,38

Mischung C/$C_x$ Rf = 0,13

**Ansprüche**

1. Verwendung von Nikkomycinen, welche

a) farblos sind, in Wasser und Pyridin sehr gut löslich sind, und welche eine positive Reaktion mit Ninhydrin, Natriummetaperjodat-Benzidin und Kalimpermanganat zeigen, und welche mit Eisen-(III)-chlorid eine Gelbfärbung ergeben ;

b) die in den Figuren 1 und 2 angegebene UV- und IR-Spektren besitzen ;

c) bei chemischem Abbau Uracil, eine Amino-hexuronsäure und eine einen Pyridinring enthaltende Aminosäure ergeben ;

d) sich bei der Papierelektrophorese als amphotere Substanz erweisen und einen isoelektrischen Punkt bei etwa pH 6 besitzen ;

e) aus Kohlenstoff, Wasserstoff, Sauerstoff und Stickstoff bestehen,

als Insektizide und Akarizide.

2. Verwendung nach Anspruch 1, enthaltend mindestens einen der Wirkstoffe mit folgender vorgeschlagener Formel

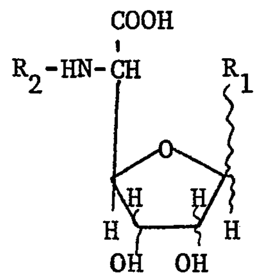

in der $R_1$ die Bedeutung

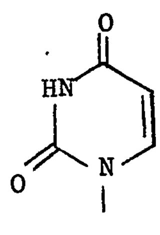

oder

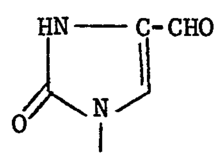

und $R_2$ die Bedeutung Wasserstoff,

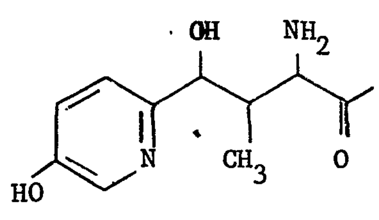

oder

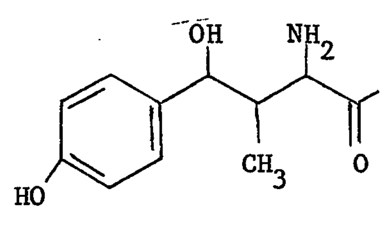

hat,

als Insektizide und Akarizide.

3. Verwendung nach Anspruch 1, enthaltend die Wirkstoffkomponentenpaare

a) 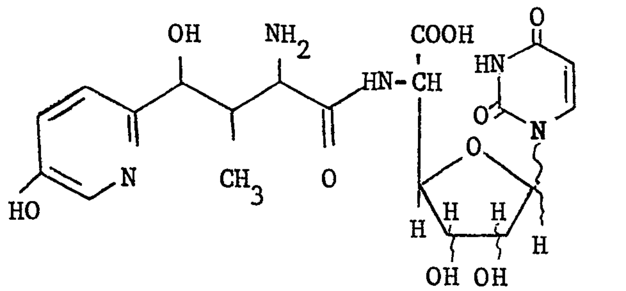 (Z) und

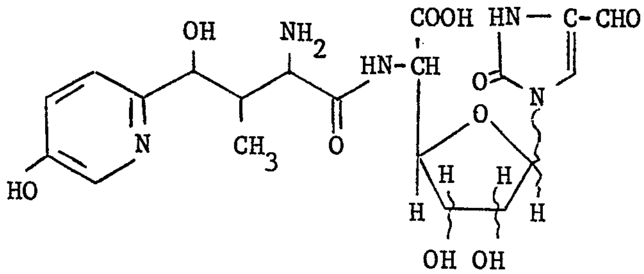 (X)

oder

b) 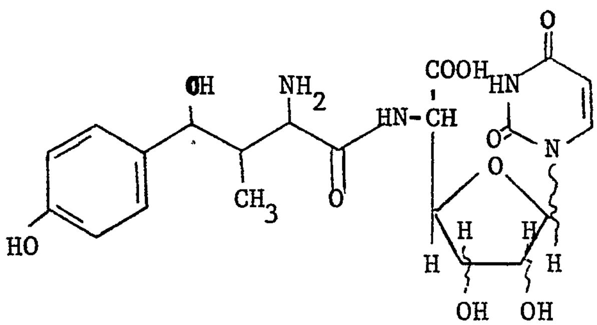 (B) und

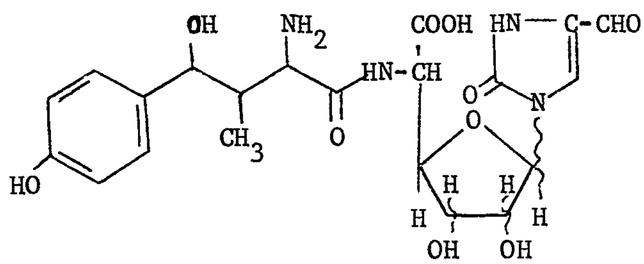 ($B_x$)

oder

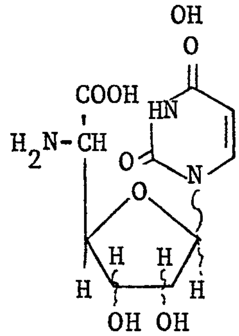 (C) und

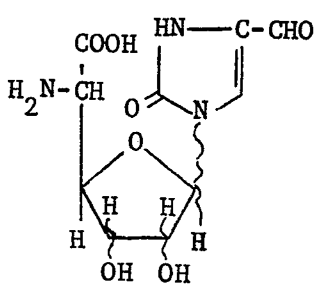 ($C_x$)

als Insektizide und Akarizide.

4. Nikkomycine der Formel

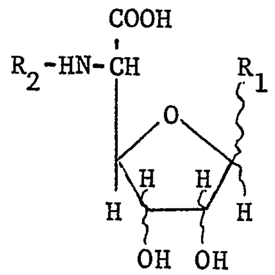

in der $R_1$ die Bedeutung

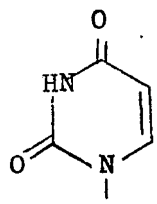

oder

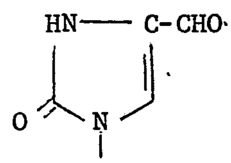

und $R_2$ die Bedeutung Wasserstoff,

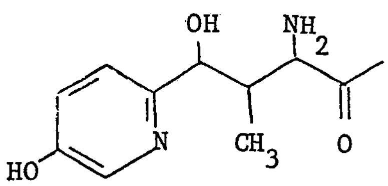

oder

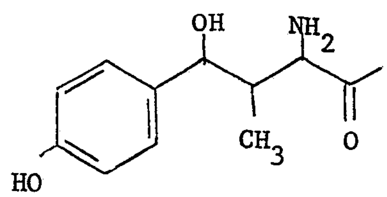

hat

mit der Maßgabe, daß, falls $R_1$ die Bedeutung

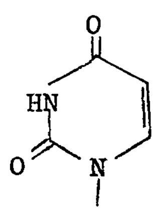

hat,

$R_2$ nicht

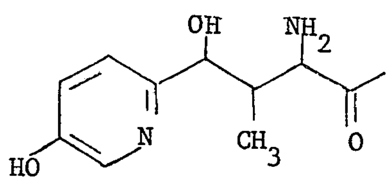

sein kann.

5. Nikkomycine nach Anspruch 4 der Formel

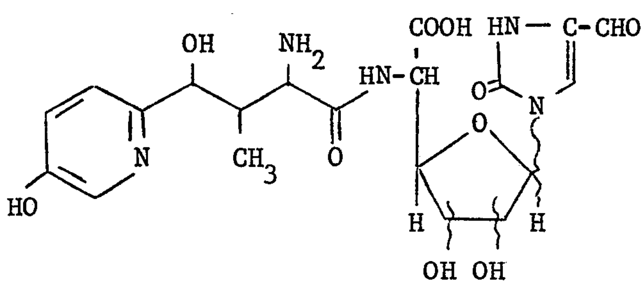

(X)

oder

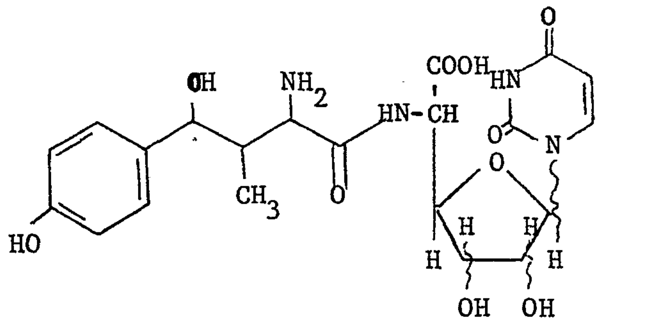

(B)

oder

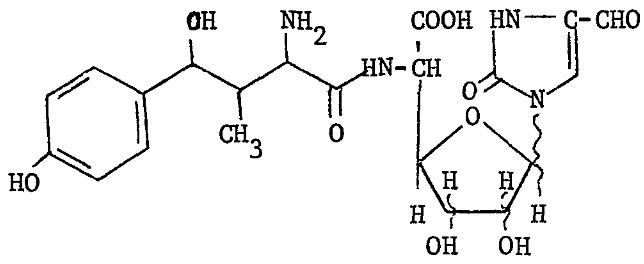

($B_x$)

6. Insektizide und akarizide Mittel, gekennzeichnet durch einen Gehalt an wenigstens einem Nikkomycin gemäß den Ansprüchen 4 und 5.

**Claims**

1. Use of Nikkomycins which

a) are colourless, are very readily soluble in water and pyridine, and which give a positive reaction with ninhydrin, sodium metaperiodate/benzidine and potassium permanganate and which give a yellow colouration with iron-III chloride ;

b) have the UV and IR spectra given in Figures 1 and 2 ;

c) on chemical degradation yield uracil, an amino-hexuronic acid and an amino-acid containing a pyridine ring ;

d) on paper electrophoresis prove to be an amphoteric substance and have an isoelectric point at about pH 6 ;

e) consist of carbon, hydrogen, oxygen and nitrogen, as insecticides and acaricides.

2. Use according to Claim 1, containing at least one of the active compounds having the following proposed formula

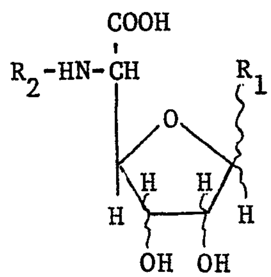

in which $R_1$ denotes

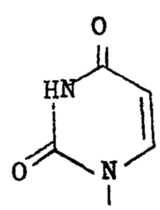

or

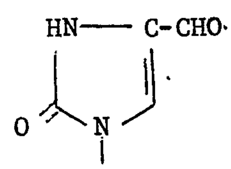

and $R_2$ denotes hydrogen,

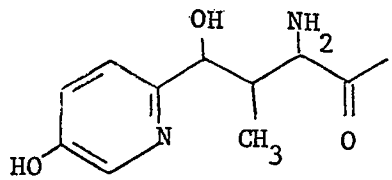

or

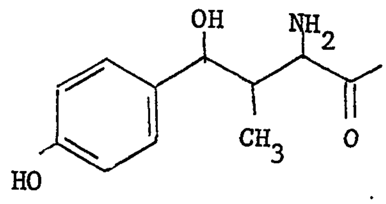

as insecticides and acaricides.

3. Use according to Claim 1, containing the pairs of active components

a)

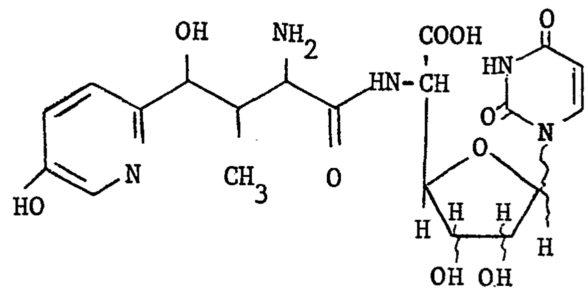

(Z) and

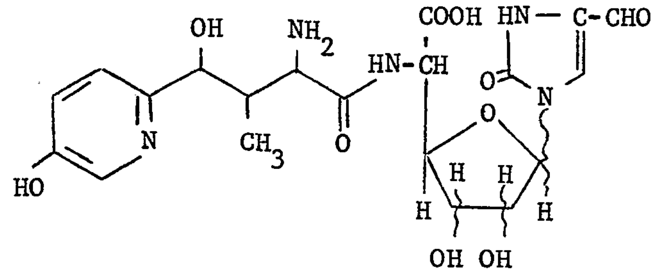

(X)

or

b)

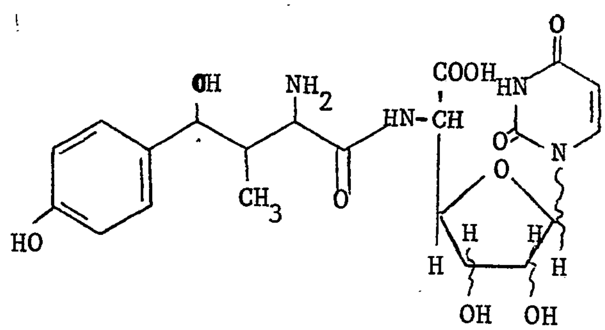

(B) and

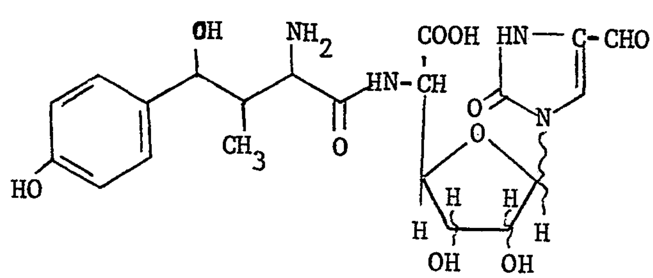

($B_x$)

or

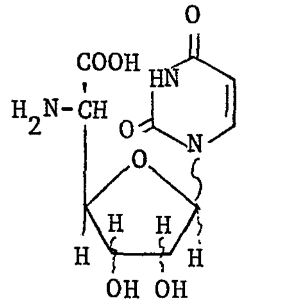

(C) and

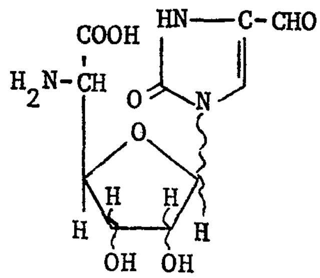

($C_x$)

as insecticides and acarides.

4. Nikkomycins of the formula

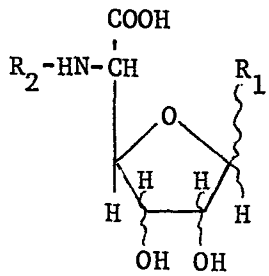

in which $R_1$ denotes

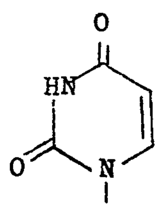

or

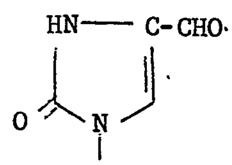

and $R_2$ denotes hydrogen,

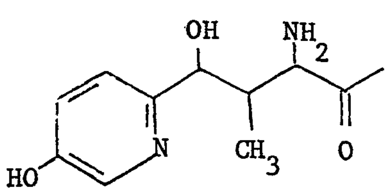

or

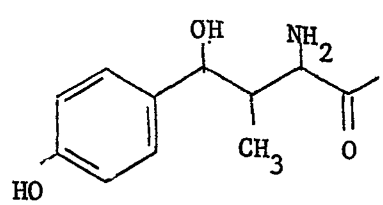

with the proviso that if $R_1$ denotes

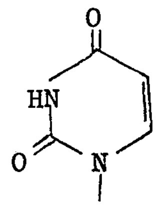

then $R_2$ cannot be

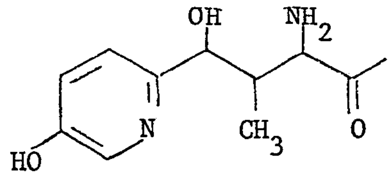

5. Nikkomycins according to Claim 4 of the formula

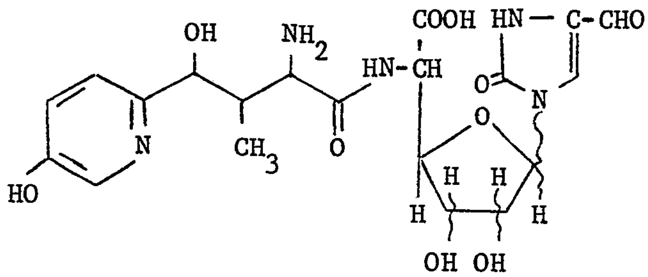

(X)

or

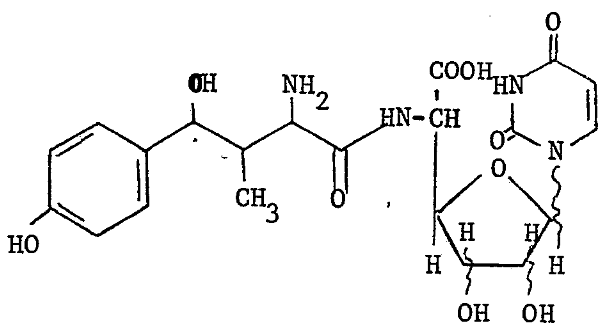

(B)

or

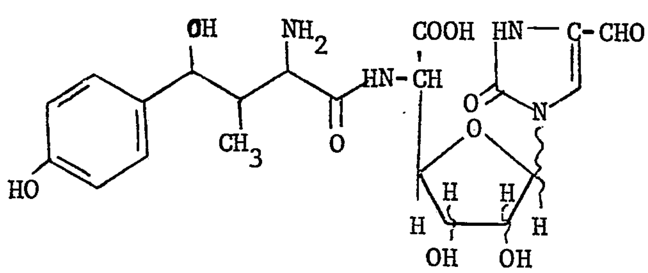

($B_x$)

6. Insecticidal and acaricidal agents characterised in that they contain at least one Nikkomycin according to Claims 4 and 5.

**Revendications**

1. Utilisation de nikkomycines,
a) incolores, très solubles dans l'eau et la pyridine, présentant une réaction positive avec la

ninhydrine, au métapériodate de sodium-benzidine et au permanganate de potassium et donnant une coloration jaune avec le chlorure ferrique ;

b) possédant les spectres ultraviolets et infrarouges reproduits dans les figures 1 et 2 ;

c) donnant par dégradation chimique de l'uracile, un acide aminohexuronique et un aminoacide contenant un cycle pyridine ;

d) se comportant comme une substance amphotère à l'électrophorèse sur papier et possédant un point iso-électrique à pH 6 environ ;

e) consistant en carbone, hydrogène, oxygène et azote, en tant qu'insecticides et acaricides.

2. Utilisation selon la revendication 1, de nikkomycines contenant au moins une des substances actives présentant la formule proposée ci-après :

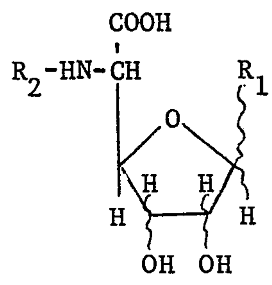

dans laquelle $R_1$ représente

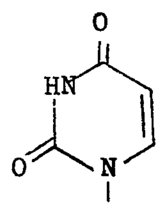

ou

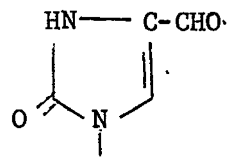

30 et $R_2$ représente l'hydrogène,

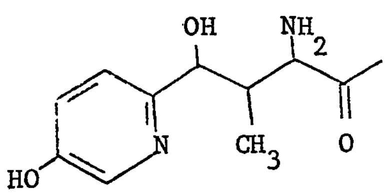

ou

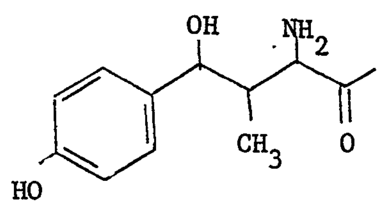

en tant qu'insectivides et acaricides.

3. Utilisation selon la revendication 1, de nikkomycines contenant les paires de composants actifs

a)

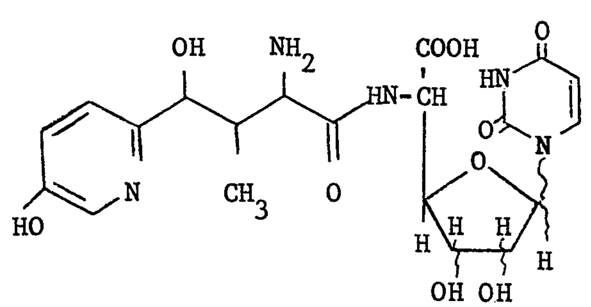

(Z) et

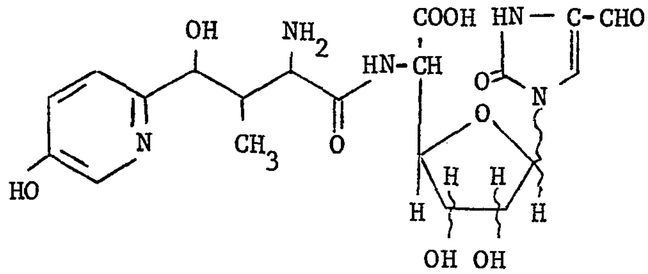

(X)

ou

b)

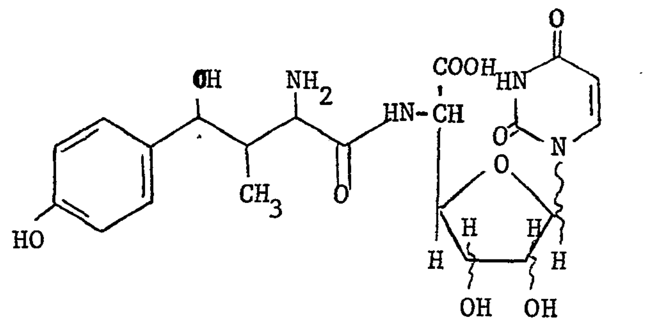

(B) et

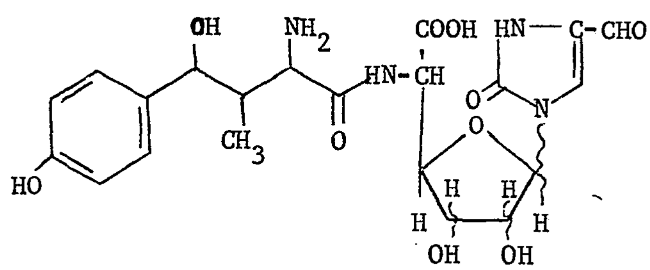

($B_x$)

ou

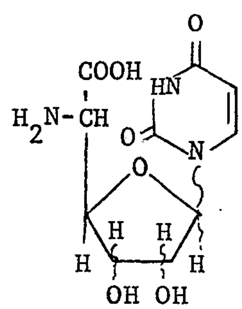

(C) et

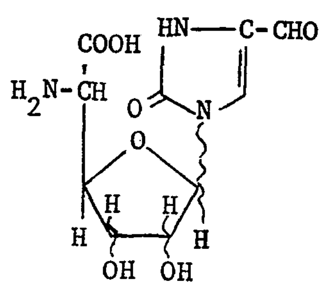

($C_x$)

en tant qu'insecticides et acaricides.

4. Nikkomycines de formule

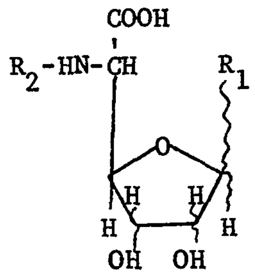

dans laquelle $R_1$ représente

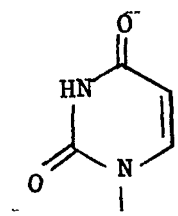

ou

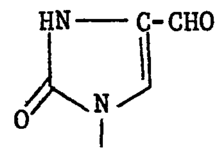

et $R_2$ représente l'hydrogène,

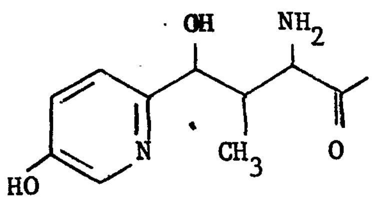

ou

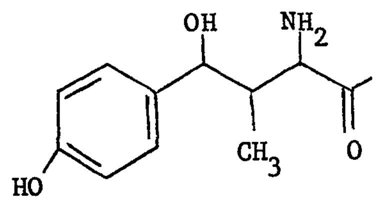

étant spécifié que lorsque $R_1$ représente

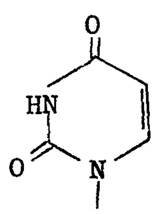

$R_2$ ne peut représenter

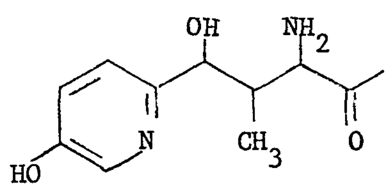

5. Nikkomycines selon la revendication 4, de formule

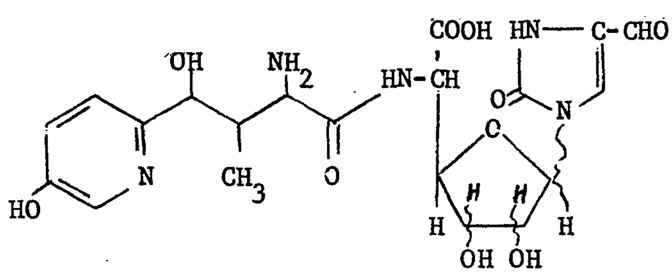

(X)

ou

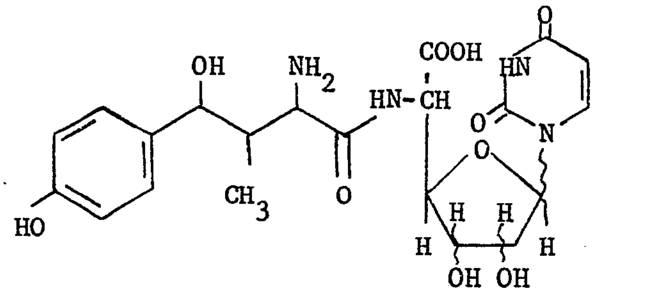

(B)

ou

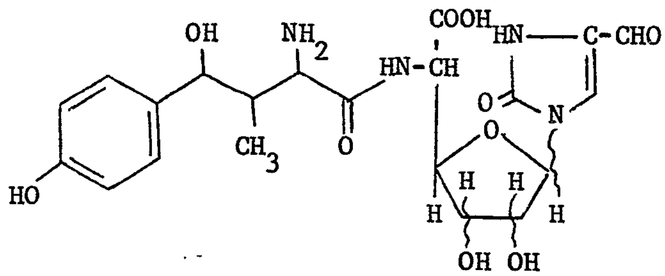

($B_x$)

6. Produits insecticides et acaricides caractérisés en ce qu'ils contiennent au moins une nikkomycine selon les revendications 4 et 5.

1/1

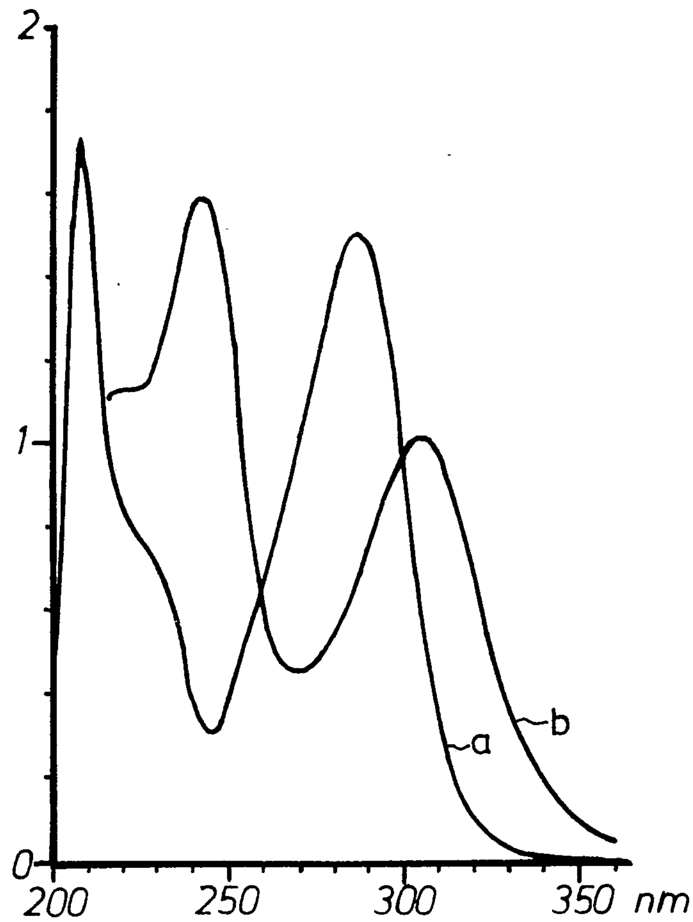
2
1
0
200
250
300
350 nm
a
b

FIG. 1

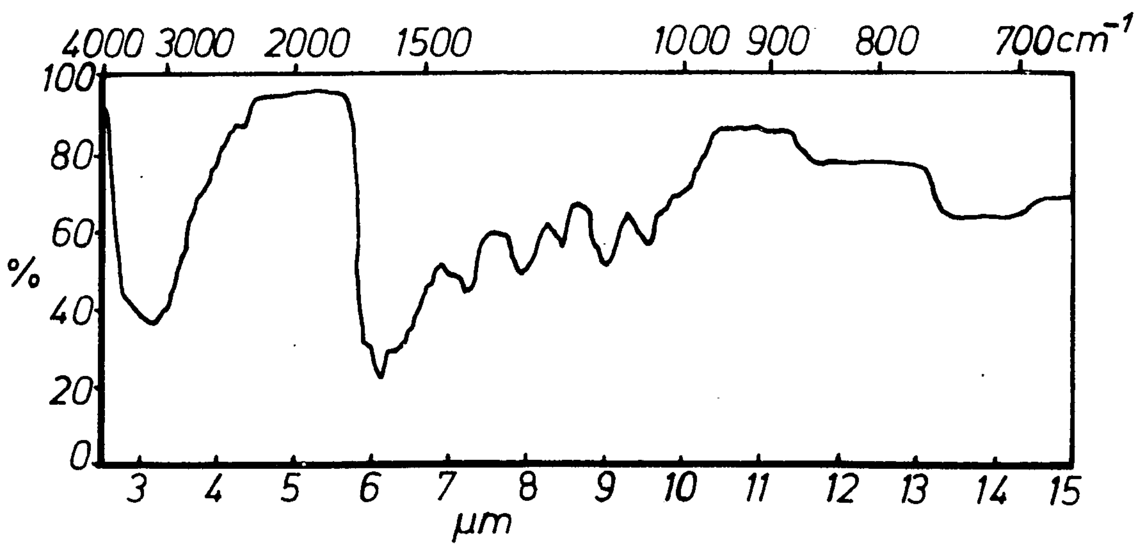
4000 3000 2000 1500 1000 900 800 700cm⁻¹
100
80
60
%
40
20
0
3 4 5 6 7 8 9 10 11 12 13 14 15
μm

FIG. 2